Europäisches Patentamt

(19) European Patent Office

Office européen des brevets

(11) Publication number: **0 172 964**
A1

# EUROPEAN PATENT APPLICATION

(12)

(21) Application number: 84305965.0

(22) Date of filing: 31.08.84

(51) Int. Cl.⁴: **A 46 B 15/00**, A 47 K 7/00,
A 61 B 19/00

(43) Date of publication of application: 05.03.86
Bulletin 86/10

(71) Applicant: **WARNER-LAMBERT CANADA INC.,**
**2200 Eglinton Avenue East, Scarborough Ontario**
**M1K 5C9 (CA)**

(72) Inventor: **Grewal, Narinder S., 64 Dundalk Drive,**
**Scarborough Ontario M1P 4T7 (CA)**
Inventor: **Nawrocki, John D., 17 Blaine Drive, Don Mills**
**Ontario M3B 2G3 (CA)**

(74) Representative: **Jones, Michael Raymond et al,**
**HASELTINE LAKE & CO. Hazlitt House 28 Southampton**
**Buildings Chancery Lane, London WC2A 1AT (GB)**

(84) Designated Contracting States: **DE FR GB IT**

(54) A surgical scrubbing device.

㉝ There is disclosed a surgical scrubbing device comprising a body portion wherein the body portion is (a) formed, at a first region thereof, with an array of bristles (17) capable of serving as a scrubbing brush and (b) formed with an extension (18) having a pointed end capable of serving as a finger-nail-cleaning pick, and wherein a sponge portion (14), suitable for holding a disinfectant detergent composition, is attached to a second region of the body portion. The device provides a one-piece unit having a nail-cleaning pick readily available to the user in pre-surgical scrup procedure.

EP 0 172 964 A1

-1-

## A SURGICAL SCRUBBING DEVICE

The present invention relates to a surgical scrubbing device and more especially to a pre-surgical scrub and nail cleaning device with a built-in nail cleaner and a sponge attachment suitable for containing an amount of disinfectant detergent.

Before engaging in surgical operations, the operating room staff, namely the surgeons and nurses, are required to follow recognized procedures for scrubbing their hands, arms and nails for a predetermined length of time in a suitable manner with acceptable disinfectant detergent. Generally, the surgical staff members have available single-use sponge-brush units containing suitable disinfectant detergent.

The present invention provides a surgical scrubbing device comprising a body portion wherein the body portion is (a) formed, at a first region thereof, with an array of bristles capable of serving as a scrubbing brush and (b) formed with an extension having a pointed end capable of serving as a finger-nail-cleaning pick, and wherein a sponge portion, suitable for holding a disinfectant detergent composition, is attached to a second region of the body portion.

This device, therefore, provides in one unit a scrubbing brush and nail pick device permitting the operating room staff to attend to careful cleaning of not only their hands and arms but also their fingernails without needing to resort to the use of separate nail-cleaning devices which might readily become misplaced or which might not be as readily subject to careful control of their sterility.

The body portion is preferably a one-piece moulding of synthetic resinous material with the bristles and extension being formed integrally with the body portion. The first and second regions are conveniently on opposite faces of the body portion.

In preferred embodiments of the present invention the extension is relatively thick and rigid as compared with the bristles, and, preferably, the body portion has, on each of two opposite sides, wall members which define, in the body portion, a channel within which the sponge portion is attached.

The extension is preferably formed with one of the wall members and, in one embodiment of the present invention, the extension may comprise a tapering prolongation on one end of the wall member, the extension preferably tapering in thickness and in width towards the remote end of the extension, the remote end of the extension preferably being rounded in side profile. In this embodiment the extension may be formed integrally on each side with a longitudinally-extending strengthening rib.

In a second embodiment of the present invention, the extension comprises an elongated handle portion and a pointed nail pick end portion extending above and longitudinally of the said one wall member, wherein the extension is connected integrally with the wall member by a plurality of readily breakable posts permitting the handle and pick portion to be detached from the wall member. In this embodiment the said one wall member is of reduced height as compared with the wall member on the opposite side.

The scrubbing devices of the present invention are preferably in a microbiologically sterile form and may be enclosed in a sealed wrapping prior to use.

For a better understanding of the present invention, and to show how the same may be put into effect, reference will now be made, by way of example, to the accompanying drawings, in which:

Figures 1, 2 and 3 show a plan view, side view, and view from beneath of one form of device in accordance with the present invention;

Figure 4 shows an enlarged view of the portion of the device circled at 4 in Figure 2;

Figure 5 shows a plan view of the portion of Figure 4;

Figure 6 shows an end view of the device;

Figure 7 shows a partial end view of the device on a larger scale;

Figure 8 shows a plan view of a second form of a device in accordance with the invention;

Figure 9 shows a side view of the device of Figure 8; and

Figure 10 shows an end view of the device of Figures 8 and 9.

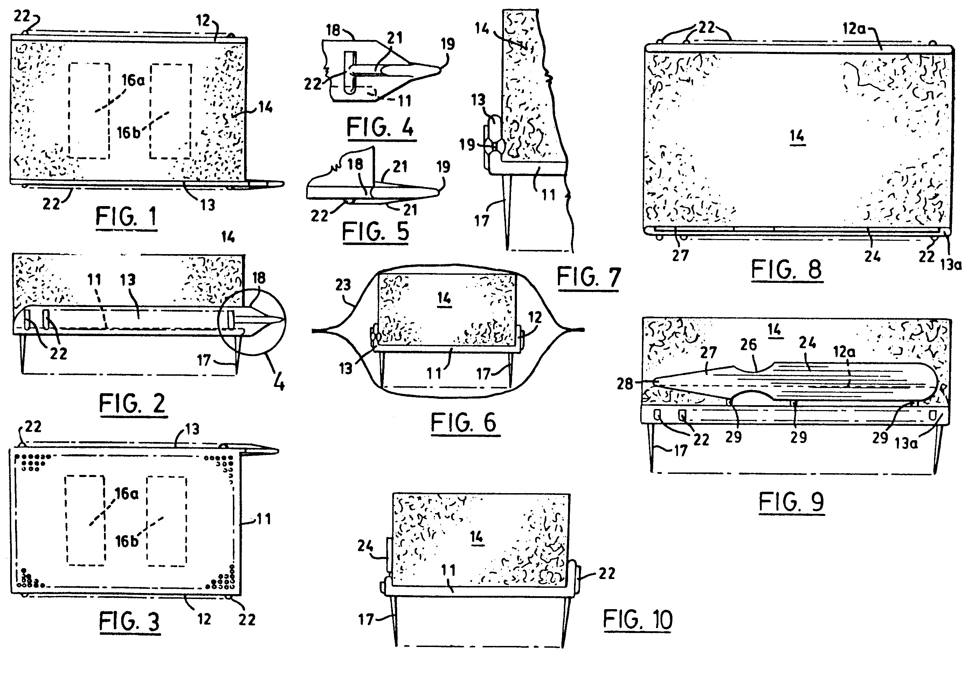

Referring to the drawings, wherein like reference numerals indicate like parts, in Figures 1 to 7 a surgical scrubbing device has a body portion which is a one-piece moulding of a synthetic resinous material e.g. polyethylene. The body portion comprises a central plate member 11, having integrally moulded on each side an upstanding wall member 12 and 13. These define, with the plate member 11, a U-shaped channel which snugly receives an open cell synthetic resinous, e.g. polyurethane, foam sponge pad 14 of rectangular shape. The lower surface of the pad 14 is bonded to the plate member 11 with an adhesive applied on the upper surface of the plate member 14 in the two rectangular areas 16a and 16b shown in broken lines in Figures 1 and 3.

As supplied to the user, the sponge pad 14 may be impregnated with a predetermined quantity of a disinfectant detergent, or the pad may be left unimpregnated, and the user may apply to the pad a disinfectant detergent solution of his choice. The plate member 11 has integrally moulded on its lower side an array of relatively thin and flexible tapering bristles 17 providing an integral scrubbing brush portion. For example, there may be about 60 rows of the bristles extending longitudinally along the lower surface of the plate member 11, each row consisting of about 30 of the bristles extending transversely across the lower surface of the plate member 11.

At one end, the wall member 13 is formed with an integral extension 18, shown in more detail in Figures 4 and 5, protruding beyond the end wall of the pad 14. As shown in side profile in Figure 4, the extension 18 tapers sharply and then more gently toward a rounded nose portion 19. As shown in plan in Figure 5, the extension 18 in the region of the nose portion 19 tapers in thickness towards its end, and terminates in a part-cylindrical end edge. The extension 18 is relatively thick and rigid as compared with the bristles 17 and, on each side, the extension 18 is formed integrally with a stiffening rib 21, the outer side of which tapers towards its end to blend smoothly with the sides of the nose portion 19.

The outer sides of the wall members 12 and 13 may be ribbed e.g. by the provision of a plurality of regularly spaced integrally-moulded ribs 22 to facilitate gripping the scrubbing device in the fingers.

The whole device may be packaged in an airtight film wrapper 23, and may be sterilised by conventional means, to provide a microbiologically sterile, sealed unit.

In use, the user employs the sponge pad 14 as an applicator to apply disinfectant detergent solution to his hands and arms, and employs the scrubbing bristles 17 to scrub up in the conventional manner. While gripping the side walls 12 and 13 of the device between the fingers of one hand, the nose portion 19 can be readily employed as a nail pick to clean the nails of the other hand.

In the form shown in Figures 8, 9 and 10, the wall member 13a on one side is formed with reduced height as compared with the wall member 12a on the opposite side and on the upper surface of the wall member 13a is integrally moulded an elongated nail pick member having a handle portion 24, an intermediate portion 26 of reduced width and an end portion 27 tapering to a part-cylindrical nose portion 28. The end portion 27 and the handle portion 24 are integrally connected to the upper side of the wall member 13a by three small posts 29. The nail pick member is again relatively thick and rigid as compared with the bristles 17 and, in use, can be detached from the device by bending it outwardly away from the pad 14 to fracture the posts 29. In this case, after applying the disinfectant detergent solution to the hands, the nail pick member can be readily manipulated with the thumb and index finger grasping the reduced width portion 26 to employ the nose portion 28 to clean under the finger nails.

With the devices illustrated in the drawings, and other embodiments of the scrubbing device, the brush portion comprising the plate member 14 and the side walls 12 and 13 or 12a and 13a, the bristles 17, and the nail pick member 18 or 24 can be readily moulded in one piece using conventional moulding techniques, e.g. injection moulding.

CLAIMS:

1. A surgical scrubbing device comprising a body portion wherein the body portion is (a) formed, at a first region thereof, with an array of bristles capable of serving as a scrubbing brush and (b) formed with an extension having a pointed end capable of serving as a finger-nail-cleaning pick, and wherein a sponge portion, suitable for holding a disinfectant detergent composition, is attached to a second region of the body portion.

2. A surgical scrubbing device according to Claim 1, wherein the body portion is a one-piece moulding of synthetic resinous material and the bristles and extension are formed integrally with the body portion.

3. A device according to Claim 1 or 2, wherein the first and second regions are opposite faces of the body portion.

4. A device according to Claim 1, 2 or 3, wherein the extension is relatively thick and rigid as compared with the bristles.

5. A device according to any preceding claim, wherein the body portion has, on each of two opposite sides, wall members which define, in the body portion, a channel within which the sponge portion is attached.

6. A device according to Claim 5, wherein the extension is formed with one of the wall members.

7. A device according to Claim 6, wherein the extension comprises a tapering prolongation on one end of the wall member, the extension preferably tapering in thickness and in width towards the remote end of the extension, the remote end of the extension preferably being rounded in side profile.

8.    A device according to any preceding claim, wherein the extension is formed integrally on each side with a longitudinally-extending strengthening rib.

9.    A device according to Claim 6, wherein the extension comprises an elongated handle portion and a pointed nail pick end portion extending above and longitudinally of the said one wall member and wherein the extension is connected integrally with the wall member by a plurality of readily breakable posts permitting the handle and pick portion to be detached from the wall member.

10.    A device according to Claim 9, wherein the said one wall member is of reduced height as compared with the wall member on the opposite side.

11.    A device according to any preceding claim, wherein the device is in a microbiologically sterile form and is enclosed in a sealed wrapping.

22  12

16a

16b

14

22  13

## FIG. 1

14

11  13  18

22

17  4

## FIG. 2

22  13

16a

16b

11

12  22

## FIG. 3

18  21

19

22

11

## FIG. 4

18  21

19

22  21

## FIG. 5

14

13

19

17  11

## FIG. 7

23

14

12

13  11  17

## FIG. 6

22  12a

14

27  24  22  13a

## FIG. 8

14

27  26  24  12a

28

17  22  29  29  29  13a

## FIG. 9

14

24

11

22

17

## FIG. 10

1 / 1

0172964

## European Patent Office

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | FR-A-2 516 768 (THE KENDALL COMPANY) * Figure 1; page 5, lines 2-21 * | 1,3-7 | A 46 B 15/00 A 47 K 7/00 A 61 B 19/00 |
| Y | | 2,11 | |
| Y | US-A-3 447 181 (D.G. COKER & J.C. LOVELESS) * Figures; claim 1 * | 2,11 | |

TECHNICAL FIELDS SEARCHED (Int. Cl.4)

A 46 B
A 47 K
A 61 B
A 61 M
A 45 D

The present search report has been drawn up for all claims

| Place of search | Date of completion of the search | Examiner |
|---|---|---|
| THE HAGUE | 02-05-1985 | JONES T.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82